# EUROPEAN PATENT SPECIFICATION

(11) **EP 0 403 101 B1**
(45) Date of publication and mention of the grant of the patent: **21.04.1993**
(21) Application number: 90305798.2
(22) Date of filing: 29.05.1990
(51) Int. Cl.: A61F 5/453

(54) **Retractile penis device**
Vorrichtung für einen zurückziehbaren Penis
Dispositif pour un pénis rétractile

(30) Priority: 12.06.1989 GB 8913480
(43) Date of publication of application: 19.12.1990
(73) Proprietor: E.R. SQUIBB & SONS, INC., Princeton New Jersey 08543-4000 (US)
(72) Inventor: Steer, Graham Emery, Fulham, London SW6 6AF (GB); Auld, David John c/o D. Young & Co., London WC1V 7RD (GB)
(74) Representative: Cook, Anthony John

(56) References cited:
- GB-A- 1 274 374
- US-A- 4 588 397

## Description

Urinary incontinence can result from a variety of physical or mental dysfunctions. For men this problem can be coped with by employing an indwelling catheter or more commonly in ambulatory patients by use of an external catheter which is adhered to the shaft of the penis by a double sided adhesive strip. Such type of system is shown, for example, by Rogers et al. in United States Patents 3,835,857 and 3,863,638.

Another approach involves an external catheter in combination with special constructed briefs or harness. Such systems are shown, for example, by Stein in U.S. Patent 4,022,213, by Komis in U.S. Patents 4,553,968 and 4,713,066, by Giacalone in U.S. Patents 4,568,340 and 4,588,397, by Jensen et al. in U.S. Patent 4,673,401, by Smith in U.S. Patent 4,813,943, by Matsuura in U.S. Patents 4,838,883 and 4,886,510, by Terauchi et al. in U.S. Patent 4,820,291, by Salt in U.K. Patent 1,274,374, by Cross in U.K. Patent Application 2,223,173A, by Wirkner in German Offenlengungsschrift 2,727,916, by Ozenne in European Patent 32,138, and by Demoulin in European Patent 119,143.

In some instances the problem of male urinary incontinence is further complicated because the patient also is suffering from a retractile penis. Such condition occurs frequently in patients who are obese or who have lost muscle control in the lower abdomen and makes it difficult, if not impossible, to successfully attach an external catheter or to employ the male incontinence briefs described above. In many circumstances, a retractile penis is a sufficient problem to prevent normal urination even when the patient would otherwise be continent. Such patients are often forced to rely on diapers as the only way to manage their condition.

This invention relates to a device as specified in the preamble of claim 1 for use by males suffering from a retractile penis. Such a device is known, for example from British Patent (GB-A) No. 1,274,374.

According to this invention, the device is provided for use by persons suffering from a retractile penis, characterised by the rear surface of a plate which contacts the user's body being convexly curved so as to enhance the extent to which the penis projects from the body.

In a preferred embodiment of the invention, a condom-like urine channelling device has one of its ends sealingly attached to the attachment ring and the other end empties into a collection bag. The plate may be of hollow construction and contain a cushioning liquid or a gel or a plastic foam material. Alternatively, the plate can have a plastic foam backing.

The term "convex" refers to the surface of the plate which in use is applied to the skin of the user. The opposite surface of the plate carrying the attachment ring may be concave with respect to the user's abdomen or may be flat.

The apertured plate may have means thereon by which a belt or harness can be attached to the plate. In use, the harness holds the plate in towards the abdomen of the user. Alternatively, the plate may be attached to a suitable garment such as a pair of briefs having an elasticized waist.

The attachment ring preferably has a peripheral channel in its radially outer surface so that the elasticated mouth of the condom-like channelling device can be stretched over and sealingly attached to the ring.

The plate may be attached in any suitable way to a pair of briefs. For example, by stitching or by adhesive or by tabs and cooperating loops.

The following drawings illustrative of the invention:
Figure 1 is a perspective view of one example of the invention;
Figure 2 is a rear view of the device shown in Figure 1;
Figure 3 is a cross-section view of the device shown in Figures 1 and 2 along the line 2 - 2;
Figure 4 is a cross-sectional view similar to Figure 3 but showing an alternative embodiment of the invention;
Figure 5 is a perspective view showing a plate for use in another embodiment of the invention;
Figure 6 is a cross-sectional view taken in axial plane of the plate shown in Figure 5;
Figure 7 is a view similar to Figure 6 but showing the whole device;
Figure 8 illustrates a pair of briefs incorporating a device such as illustrated in Figures 5 - 7;
Figure 9 is a perspective view of briefs fitted with another embodiment of the invention;
Figure 10 is a exploded view showing the apertured pad, two part coupling, and condom-like channelling device of Figure 9;
Figure 11 is a perspective view of the apertured pad shown in Figures 9 and 10;
Figure 12 is a cross-section through the apertured pad and one element of the coupling shown in Figures 9 and 10;
Figure 13 is a perspective of another embodiment of the apertured pad of this invention with tags for attachment to a pair of briefs;
Figure 14 is a side view of the apertured pad of Figure 13; and
Figure 15 is a cross-section view through the apertured pad of Figures 13 and 14.

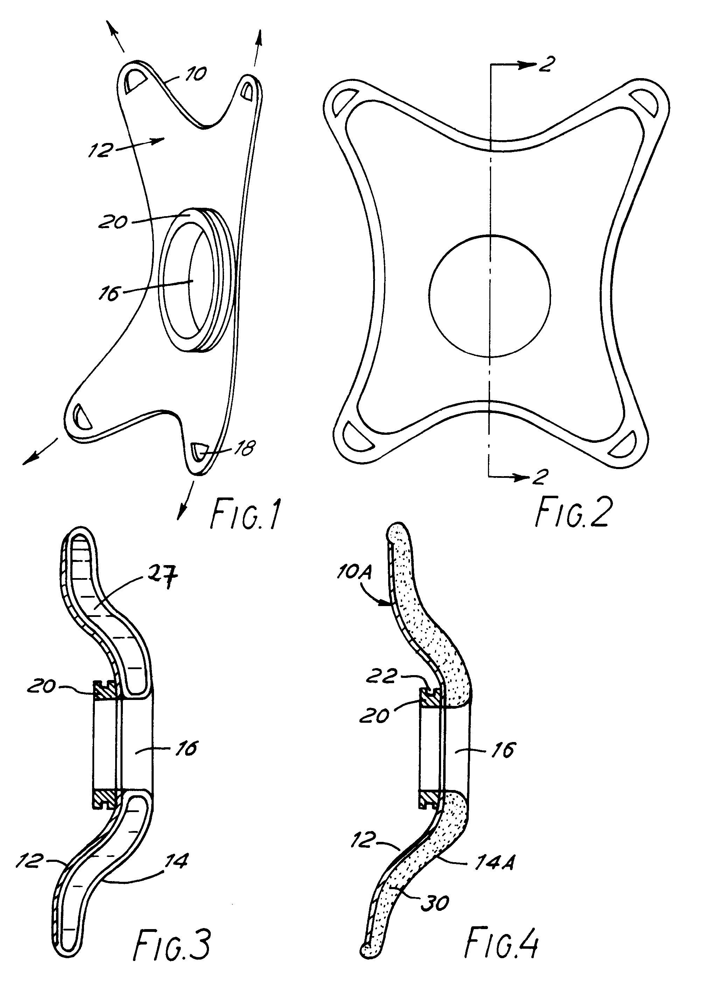

Figures 1 to 3 illustrate a first embodiment of an apertured convexly curved plate 10 and attachment ring 20 for use by a male suffering from a retractile penis. Plate 10 is of a hollow construction having a front wall 12 and a rear generally convex wall 14. The space between these walls is filled with a gel 27 such as silicone gel, for example, Dow Corning Mastectomy Gel®.

Plate 10 has a central aperture 16 for receiving a penis and is made of a flexible or semi-flexible synthetic plastics material such as polyethylene. The plate has four holes 18 which form a means whereby a belt or harness (not shown) can be attached in order to hold the plate in position on the wearer. In use, the convex surface 14 is pressed against the body of the wearer surrounding the penis and tends to enhance the extent to which the penis projects from the body. At the same time, discomfort to the wearer is minimized because the gel distributes the pressure.

The surface 12 of the plate has a ring 20 attached thereto surrounding the aperture 16. The ring 20 is also formed of a synthetic plastics material such as polyethylene which may be semi-flexible to the same or less extent than plate 10. The materials of ring 20 and plate 10 are selected so that ring 20 can be secured to surface 12 by heat, ultra sound, adhesives, or by other conventional methods of attaching plastics. Ring 20, as shown in Figure 4, has a peripheral channel 22 intended to receive an elasticated rim 24 of a condom-like channelling device 26 as shown in Figure 7.

The channelling device 26 is formed of flexible plastic or natural material such as polyethylene or latex rubber with a narrow outlet 28 at the end opposite elasticated rim 24. If the user is incontinent, then opening 28 would be connected to a bag worn by the user on his leg. An example of a suitable connector means is shown by Peter L. Steer et al. in United States Patent 4,770,445. Also, the channeling device can include a one way valve near the elasticated rim to prevent the backflow of urine onto the penis of the incontinent user.

Figure 4 illustrates an alternative embodiment of the invention where plate 10A is not hollow but instead front wall 12 of flexible or semi-flexible plastics material has a foam pad 30 secured thereto. Foam pad 30 has a rear convex surface 14A which is of the same configuration has rear wall 14 of plate 10 shown in Figures 1 to 3.

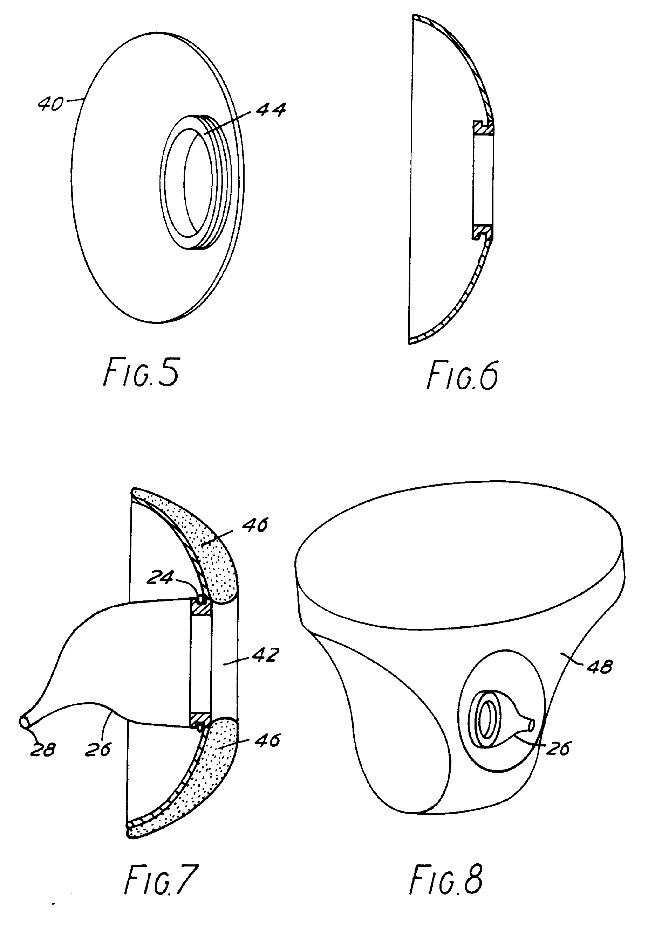

Figures 5 to 8 show an embodiment having a generally spherical plate 40 of semi-rigid synthetic plastics material having a central aperture 42. An attaching ring 44 is shown secured to the circumferential surface of aperture 42. The opening of ring 44 is somewhat narrower than opening 42 but is still large enough to accomodate the user's penis. Ring 44 is provided with a channel identical to channel 22 of ring 20 to which the elasticated rim 24 of channelling device 26 is secured. A foam pad 46 is secured to plate 40 surrounding aperture 42 as shown in Figure 7. Again, the outer surface of foam pad 46 has a convex configuration. Plate 40 is attached by stitching or by adhesive to a pair of briefs 48 which may be wholly or partly formed of elasticated material.

Foam pads 30 and 46 can be made from any suitable polymeric material such as a closed cell or semi-open cell polyurethane or polyethylene foam. Foam pad 30 can be secured to plate 10A and foam pad 46 can be secured to plate 40 by means of adhesive.

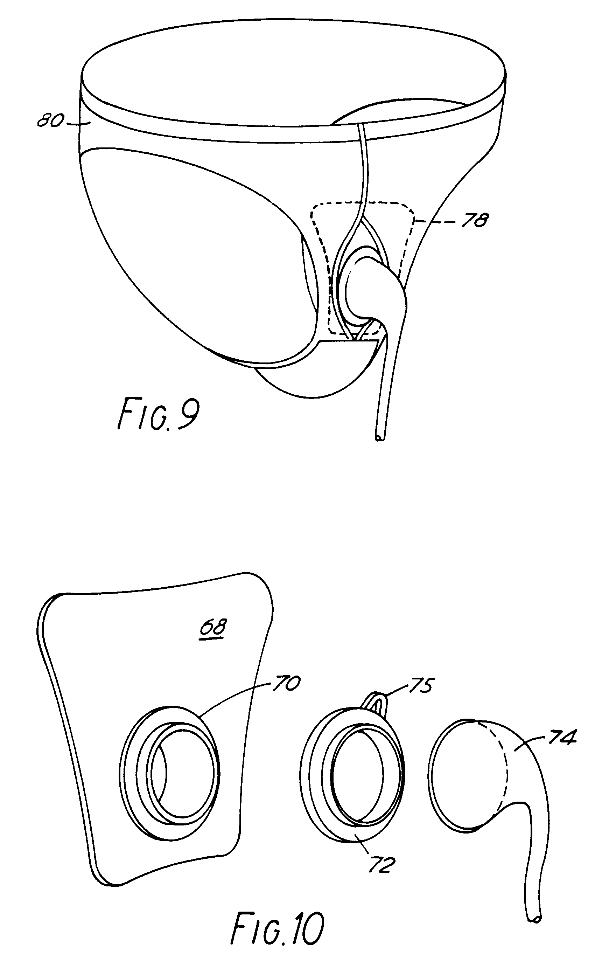

Figures 9 to 12 show an embodiment of this invention comprising an apertured pad 60 having a convex rear surface 62 and an aperture 64 intended to receive or surround a partly retracted penis. Pad 60 is constructed of a silicone rubber skin 66 filled with a silicone gel or a low density polymeric foam 69. The front surface 68 of pad 60 opposite convex rear surface 62 is preferably substantially flat. One coupling element 70 of a two-piece snap-fit coupling is secured to front surface 68 around aperture 64 by adhesive or other suitable means. The second coupling element 72 is dimensioned to snap over coupling element 70 and trap one end of a condom-like channelling device 74 between the two coupling elements. Coupling element 72 includes a pull tab 75 to aid in separating the coupling elements. Coupling elements 70 and 72 are molded from a semi-rigid synthetic plastics material such as polyethylene and channelling device 74 is formed a thin flexible synthetic plastics or natural material such polyethylene or latex rubber. As with channelling element 26, if the patient is incontinent element 74 would be provided at the discharge end with a coupling member that connects to a leg bag. If the patient is continent, then the user can remove the second coupling element and the channelling device and urinate in a normal manner.

The pad 60 is disposed of within a pocket 78 in a pair of briefs 80 which preferably are made of two-way stretch highly elastic material. The pad may be maintained within the pocket by one or more fabric fasteners of the type having interengaging hooks and loops. Velcro® strips may be used for this purpose.

An advantage of employing the apertured pad of the embodiment of Figures 9 to 12 is that the pad presents a smooth and comfortable surface to the wearer that seals effectively against the wearer's skin at the area close to or surrounding the base of the penis. Also, the readily deformably nature of filler 69 means that painful pressure points or pressure sores are unlikely to occur. Further, the two part coupling provides the wearer with a quick and easy method of urinating normally if he wishes.

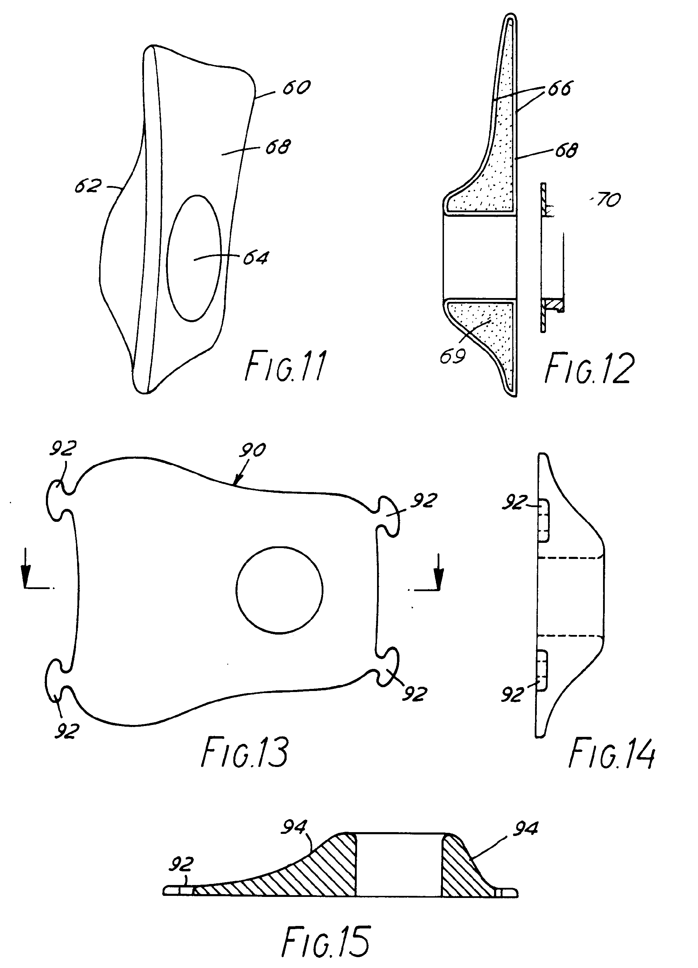

A further alternative embodiment of the invention is illustrated in Figures 13 to 15. This embodiment is similar to that of Figures 9 to 12 but provides the apertured pad 90 with four tabs 92, one at or near each corner. These tabs are intended to cooperate with suitable loops or slots positioned within a pair of briefs. This eliminates the need for a pocket within the briefs so that a wholly smooth surface 94 is presented towards the wearer's skin rather than a fabric pocket retaining wall as in the embodiment of Figure 9. This is advantageous because the stitching needed to connect the fabric wall to the remainder of the briefs can be a cause of irritation and result in pressure sores when the device is worn for long periods of time.

## Claims

1. A device for use by a person suffering from a retractile penis comprising a plate (10,40,60) having an aperture (16,42,64) for receiving the user's penis, an attachment ring (20,44,70) secured to said plate about said aperture, a condom-like urine channelling device (26,74) sealingly attached to said ring, characterized in that the rear surface (14,14A,62,94) of said plate which contacts the user's body is convexly curved so as to enhance the extent to which the penis projects from the body.

2. A device according to Claim 1 in which the plate is of hollow construction and contains a cushioning material between the front and rear walls of the plate.

3. A device according to Claim 1 in which the plate is a single wall having a foam pad (46) secured to it, the wall and pad being curved so that the convexly curved pad surface contacts the user's body.

4. A device according to Claim 1 in which the attaching ring has a peripheral channel (22) and the condom-like channelling device has an elasticated rim (24) which is stretched over the attaching ring and is received within said peripheral channel.

5. A device according to Claim 2 in which the front wall of the plate is substantially flat.

6. A device according to Claim 5 in which the attaching ring comprises a first coupling element (70) secured to the flat front wall of said plate around the aperture and a second coupling element (72) which is a snap fit over the first coupling element, and wherein one open end of the condom-like channelling device is trapped between the first and second coupling elements.

7. A device according to Claim 1 in which the apertured plate has means (18,92) thereon by which a belt or harness can be attached to the plate or by which the plate can be secured to briefs (48,80).

## Patentansprüche

1. Vorrichtung für eine Person, die an einem zurückziehbaren Penis leidet, mit: einer Platte (10, 40, 60), die eine Öffnung (16, 42, 64) zum Aufnehmen des Penis des Benutzers aufweist, einem Befestigungsring (20, 44, 70), der an der Platte um die Öffnung herum befestigt ist, einem kondomähnlichen Urinableitungsgerät (26, 74), das an dem Ring abgedichtet befestigt ist, dadurch gekennzeichnet, daß die rückwärtige Oberfläche (14, 14A, 62, 94) der Platte, die den Körper des Benutzers berührt, konvex gebogen ist, um so die Länge, bis zu der der Penis vom Körper vorragt, zu erhöhen.

2. Vorrichtung nach Anspruch 1, wobei die Platte aus einer Hohlkonstruktion besteht und zwischen ihren Vorder- und Rückwänden ein abfederndes Material enthält.

3. Vorrichtung nach Anspruch 1, wobei die Platte eine einzelne Wand ist, die ein an ihr befestigtes Schaumkissen (46) aufweist, wobei die Wand und das Kissen so gebogen sind, daß die konvex gebogene Kissenoberfläche den Körper des Benutzers berührt.

4. Vorrichtung nach Anspruch 1, wobei der Befestigungsring einen peripheren Kanal (22) und das kondomähnliche Ableitungsgerät eine elastische Krempe (24) aufweist, die über den Befestigungsring gespannt und in dem peripheren Kanal aufgenommen ist.

5. Vorrichtung nach Anspruch 2, wobei die Vorderwand der Platte im wesentlichen flach ist.

6. Vorrichtung nach Anspruch 5, wobei der Befestigungsring aufweist: ein erstes Kupplungselement (70), das an der flachen Vorderwand der Platte um die Öffnung herum befestigt ist, und ein zweites Kupplungselement (72), das eine Schnappanpassung über dem ersten Kupplungselements ist, und wobei ein offenes Ende des kondomähnlichen Ableitungsgerätes zwischen dem ersten und zweiten Kupplungselementen eingeklemmt ist.

7. Vorrichtung nach Anspruch 1, wobei die durchbrochene Platte eine Einrichtung (18, 92) daran aufweist, mittels der ein Gürtel oder ein Geschirr an der Platte befestigt oder die Platte an Unterhosen (48, 80) befestigt werden kann.

## Revendications

1. Dispositif utilisable par une personne affligée d'un pénis rétractile, comprenant une plaque (10,40,60) percée d'une ouverture (16,42,64) de réception du pénis de l'utilisateur, une bague de fixation (20,44,70) fixée à ladite plaque autour de ladite ouverture, un dispositif genre condom (26,74) de canalisation de l'urine fixé de manière étanche à ladite bague, caractérisé en ce que la surface postérieure (14,14A,62,94) de ladite plaque qui touche le corps de l'utilisateur a une courbure convexe de manière à augmenter la distance dont le pénis dépasse du corps.

2. Dispositif selon la revendication 1, dans lequel la plaque a une structure creuse et contient une matière de rembourrage entre ses parois antérieure et postérieure.

3. Dispositif selon la revendication 1, dans lequel la plaque est une simple paroi à laquelle est fixé un tampon de mousse (46), cette paroi et ce tampon étant incurvés de telle sorte que la surface convexe du tampon touche le corps de l'utilisateur.

4. Dispositif selon la revendication 1, dans lequel la bague de fixation comporte une gorge périphérique (22) et le dispositif de canalisation genre condom comporte un rebord élastique (24) qui se tend sur la bague de fixation et se loge dans ladite gorge périphérique.

5. Dispositif selon la revendication 2, dans lequel la paroi antérieure de la plaque est pratiquement plate.

6. Dispositif selon la revendication 5, dans lequel la bague de fixation comprend un premier élément de raccord (70) fixé à la paroi antérieure plate de ladite plaque autour de l'ouverture et un second élément de raccord (72) qui s'encliquète sur le premier élément de raccord, et dans lequel une extrémité ouverte du dispositif de canalisation genre condom est emprisonnée entre les premier et second éléments de raccord.

7. Dispositif selon la revendication 1, dans lequel la plaque à ouverture porte des moyens (18,92) permettant de fixer à la plaque une ceinture ou un harnais, ou bien de fixer la plaque à un slip (48,80).
